# EUROPEAN PATENT APPLICATION

(11) **EP 1 522 323 A1**
(43) Date of publication of application: **13.04.2005**
(21) Application number: 04023893.3
(22) Date of filing: 07.10.2004
(51) Int. Cl.: A61M 1/16, A61M 1/34

(54) **Device for eliminating carbon dioxide from the blood and a crrt apparatus provided with said device**

(30) Priority: 09.10.2003 IT FI20030256
(71) Applicant: Medical Service S.r.l., 84131 Salerno (IT)
(72) Inventor: Caramuta, Angela, 84126 Salerno (IT)

(57) **Abstract**

Device for eliminating carbon dioxide from blood which can be used, in particular, for CRRT therapies, characterized in that it consists of a hollow casing (70) featuring a first inlet (74) for the oxygen under pressure, a second inlet (79) for the blood to be treated, a first outlet (75) for the blood which has already been treated and a second outlet (741) for the carbon dioxide to be eliminated, said device comprising a decapneization section (71) and a haemo-concentrator (762).

## Description

The present invention relates to a device for eliminating carbon dioxide from blood and to a CRRT equipment provided with this type of device.

The device of the present invention can be used not only with an equipment for therapeutic treatments such as those known with the abbreviation CRRT, that is to say Continous Renal Replacement Therapy.

As concerns the CRRT, it is well known that for various reasons it may be necessary to integrate or to replace the functions of the kidney in patients, so as to remove liquids and soluble waste substances, for example substances which have been given to the patient and/or waste substances which are present in blood due to surgical or pathological causes and so on. On this purpose, it is possible to adopt various procedures such as haemodialysis, haemofiltration and ultrafiltration. All these procedures consist in drawing waste products from the patient; in practice, the patient's blood is passed through filters or membranes so as to eliminate the waste substances and then reintroduced into the patient. For patients in grave conditions, procedures include Continous Arterio Venous Haemofiltration (CAVH), Continous VenoVenous Haemofiltration (CVVH), or in general, CRRT, as the abbreviation specified above. In this type of procedure, the patient is constantly connected to the machine which performs haemofiltration for a prolonged period of time.

Possible embodiments of a well-known machine used for CRRT treatments are described in US-5,211,849 and in US-6,349,170. In practice, a machine for CRRT treatments consists of a central unit which can be connected at the inlet and at the outlet with respect to a patient, for example by means of one or more catheters inserted in corresponding blood vessels so as to continuously draw the blood to be treated and to reintroduce it after treatment. The central unit of a machine for CRRT treatments usually consists of a blood pump, blood heating and processing means, such as, heparin adding means, means for feeding refill liquid into the blood, and a haemofilter. The blood which is continuously drawn from the patient is pushed along the machine circuit by means of the blood pump, so as to add heparin and reintegrating liquid properly warmed up to it ad to filter it before reintroducing it into the patient.

One of the inconveniences of traditional systems arises from the fact that they are very invasive and relatively complex.

Another inconvenience is connected with the relatively large amount of blood necessary to carry out the so called "Priming", that is to say the amount of blood circulating in the equipment.

A further inconvenience of the CRRT systems used at present arises from the fact that they present a treatment speed which is not sufficiently elevated in comparison with the patient's needs.

Another further inconvenience of well known CRRT systems arises from the fact that it is necessary to use a pump for the ultrafiltrate; this can lead to a reduced treatment tolerance in chronic or haemodynamically unstable patients, in which the refilling capacity is always unknown.

Moreover, in some cases it may be necessary to combine extrarenal therapy with a replacement respiratory therapy during which it is necessary to integrate the patient's blood saturation in oxygen concentration by removing carbon dioxide from the blood itself. By means of the haemofiltration machines used at present it is not possible to carry out a replacement respiratory therapy; for this reason, the patient needs to be connected to an independent oxygenator with obvious inconveniences caused by the cumbersomeness of this object and by an uncomfortable situation for the patient.

The main aim of the present invention is to eliminate the above mentioned inconveniences.

These results have been achieved according to the invention thanks to the idea of building a device for eliminating carbon dioxide as well as a corresponding equipment having the features described in the independent claims.

The present invention is advantageous basically thanks to the fact that by means of the device subject of the present invention it is possible to improve decapneization and to obtain a remarkable decrease in the amount of carbon dioxide in blood by means of a device which is extremely simply to use and, for this reason, with very few error possibilities for operators.

Further advantages are offered by a reduced priming value, by a very limited possibility of modifying electrolytic parameters, by the highest safety degree as regards the danger of a passage of microbubbles toward the patient thanks to the presence of a haemoconcentrator inside the device subject of the present invention.

Thanks to the present invention , the patient can undergo both an extrarenal and a replacement respiratory therapy by means of a device in which a haemoconcentrator is integrated with a decapneizator.

Moreover, the small dimensions of this device considerably facilitate its use.

A further advantage arises from the fact that the principle of the present invention can be applied to already existing machines with modifications which allow a more complete and effective use of the already existing material. Moreover, this application is also possible in clinical cases which now can only be treated by means of extremely invasive techniques; in addition, the device and the equipment provided with the device itself, in the long run, keep their features unchanged, even in case of limited maintenance.

These and other advantages and characteristics of the invention will be best understood by anyone skilled in the art from a reading of the following description in conjunction with the attached drawings given as a practical exemplification of the invention, but not to be considered in a limitative sense, wherein:
- Fig. 1 shows a possible embodiment of a CRRT equipment which uses a device according to the present invention, in which no scale details, but only schematical details are shown;
- Fig. 2 shows a possible embodiment of the device of the invention in a schematical longitudinal section view.

In the enclosed drawings a CRRT equipment according to the present invention has been marked with 1 as a whole. Equipment 1, at the inlet, is connected to a patient P by means of a first duct 9 which, for example, can be a 11,5 Fr. femoral catheter of the type DLC600 KC or of the type DLC 800 KC manufactured by Horizon Medical Product. The connection to the patient can obviously be carried out by using different material and the data concerning the other components described below are to be regarded as examples and do not limit possible alternatives.

Catheter 9 is connected to a duct 90 on which a blood pump 3 is positioned and acts downstream; moreover, on duct 90 is located a pressure gage 20 for measuring the intake arterial pressure of the patient. The sections through which the blood subsequently passes along its path are defmed by duct portions indicated by 91, 92, 93 and 94.

Pump 3 pushes the blood downstream (whose direction of flow is indicated by the arrows) which reaches a connection element 4 after heparin has been added to it by means of a duct 50 which is conveyed into duct 91 and connected to a corresponding tank 5 containing this substance (represented by syringe 5). A pressure gage 40 for measuring the pressure at that point of the path is located adjacent to connection element 4.

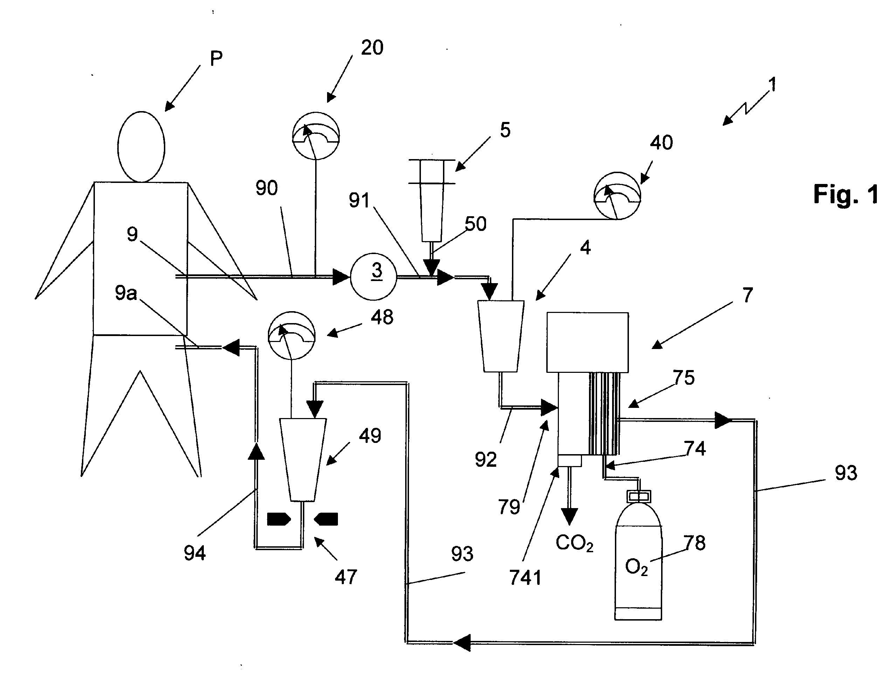

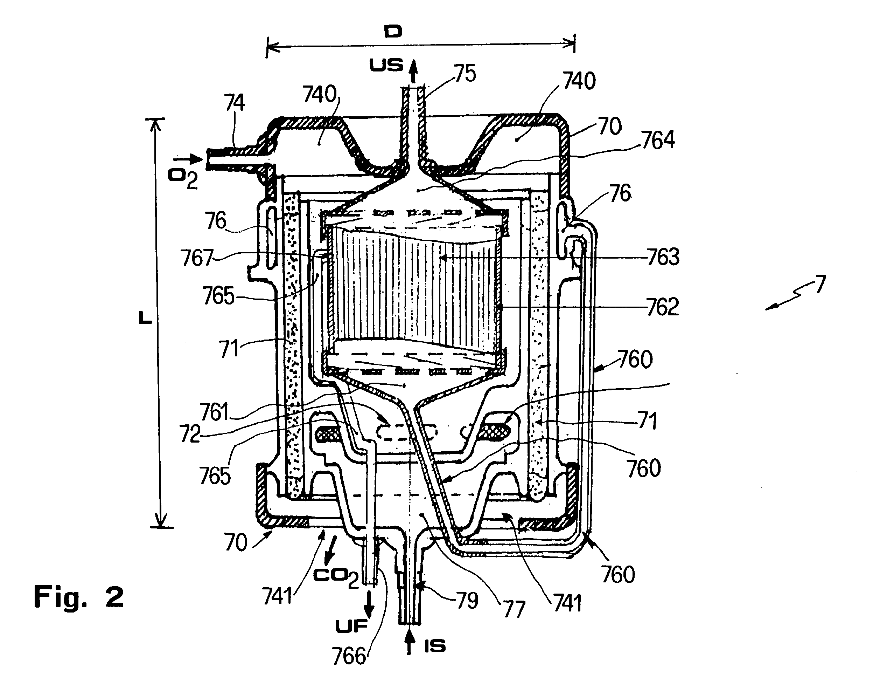

Downstream connection element 4, duct section 92 reaches an oxygenator 7 or decapneizator which is schematically illustrated in Fig 1 and is more visible in Fig. 2.

Oxygenator 7 is connected to an oxygen tank 78 ( bottle 78 O₂) and provided with an outlet 741 through which CO₂ is eliminated, that is to say conveyed toward corresponding measuring means which are not illustrated.

With a particular reference to Fig 2, the device 7 which is used for eliminating carbon dioxide consists of an external casing 70, which can be made of rigid polyurethane. External casing 70 presents a first inlet opening 74 for the oxygen, which is positioned in the upper part, a second inlet 79 for the blood to be treated which is positioned in the lower part, a first outlet 75 for the decapneized blood positioned on the upper part, a second outlet 741 for carbon dioxide and a third outlet 766 for the ultrafiltrate. Fig. 2 shows the flows of the above fluids indicated by the arrows and, in particular, IS indicates the inlet flow of the blood to be treated, US indicates the outlet flow from the device, O₂ indicates the inlet flow of oxygen, CO₂ indicates the outlet flow of carbon dioxide and UF indicates the outlet flow of the ultrafiltrate.

Inside casing 70, the blood entering from inlet 79 reaches a basically truncoconical chamber 77 positioned above said inlet 79 and equipped with a plurality of passage holes 72 which are arranged circumferencially and allow the blood to flow outwards in the radial direction. Outside holes 72 a plurality of hollow fibres 71 in polypropylene define a sort of barrier; the fibres can be wound as a skein or arranged parallel to one another. Fibres 71 present a surface substantially comprised between 0,6 and 0,8 m² and are joined by means of a potting in polycarbonate. The oxygen entering from opening 74 is blown through upper chamber 740 into fibres 71 with a flow which is preferably of 3 liters / hour. In this way, outside the fibres the blood flows at a rate of about 250-350 ml/min so as to cause haemoglobin to release carbon dioxide and to acquire oxygen. The blood will be properly diluted before having access to polypropylene fibres 71.

Around fibres 71 is arranged an annular chamber 76 entering a duct 760 which is joined to inlet section 761 of a haemo-concentrator 762 positioned inside casing 70. In particular, haemo-concentrator 762 forms a unit with the decapneizator and consists of a hollow body which is basically cylindrical and can be made of polyurethane containing hollow fibres 763 made of high permeability polysulfone. Hollow fibres 763 are arranged parallel to one another and are joined together by means of a potting in polycarbonate. The blood flowing through fibres 763 (blood which is diluted and decapneized at the haemo-concentrator inlet) causes the removal of excess water, so that the blood is returned to the patient with the correct concentration.

Haemo-concentrator 762 is also equipped with an outlet section 764 connected to said outlet 75 for the blood already treated and also with an outlet 767 connected to a discharge duct 765 leading to said outlet 766 for the ultrafiltrate.

The released carbon dioxide is expelled from device 7 through opening 741 as its passage is hindered above fibres 71.

Downstream to decapneizator 7, the section 93 of the duct reaches an element 49 consisting of a venous tank or of a device called "bubble capture device" or "bubble trap", on which a pressure gage 48 for measuring the return pressure of the patient's blood is positioned.

Downstream to element 49 of section 94 of the duct, on which an air detector is located in order to prevent possible emboli (it can be of the type called UABD, ultrasonic air bubble detector) and can be connected to a return catheter 9a which takes the blood back to patient P.

In practice, device 7 used for eliminating carbon dioxide from blood according to the present invention can be employed in a CRRT system and it has proved particularly effective in therapies which do not provide renal purification.

A non limitative application of the invention is represented by the cases of serious respiratory distress , in which the other organs are not affected.

By means of device 7 inserted in a common CRRT equipment, the amount of replacement liquid necessary for proper functioning of the decapneization section (fibres 71, that is to say CO₂ remover) can be eliminated by haemo-concentrator (section 762 of device 7).

For example, when using the device, the operator can program a CVVH with a QB superior to 250 ml/min and with an exchange frequency of about 20-30 ml/min.

As regards dimensions, casing 70 of the device presents a basically cylindrical structure with an height (L) between 5 and 20 cm and a diameter (D) between 3,5 and 15 cm.

In particular, the casing presents a height (L) of about 10 cm and a diameter (D) of about 7 cm. These dimensions are extremely small and innovative in comparison with those of traditional devices and allow the use of device 7 which can be hung it without a support element.

A further advantage of the present invention in comparison with conventional systems arises from the fact that it is much simpler than these systems because it does not require a heat exchanger and other devices which are necessary in a conventional technique.

The fact that the blood flows from a downward direction to an upward direction, both in the device section where decapneization takes place and in the haemo-concentrator section, is extremely important and provides a more complete elimination of carbon dioxide and an optimal blood treatment.

## Claims

1. Device for eliminating carbon dioxide from blood which can be used, in particular, for CRRT therapies, **characterized in that** it consists of a hollow casing (70) featuring a first inlet (74) for the oxygen under pressure, a second inlet (79) for the blood to be treated, a first outlet (75) for the blood which has already been treated and a second outlet (741) for the carbon dioxide to be eliminated, said device comprising a decapneization section (71) and a haemo-concentrator (762).

2. Device according to claim 1 **characterized in that** said decapneization section consists of a first plurality of fibres (71) which define a permeable barrier and are arranged between the inlet (79) and the outlet (75) for the blood and the position of which is such that they are flooded by the oxygen flow entering the above first inlet (74), while the blood passes through them along its path between said second inlet (79) and said outlet (75) for the blood.

3. Device according to claim 2 **characterized in that** said fibres (71) are hollow fibres in polypropylene.

4. Device according to claim 2, **characterized in that** said fibres (71) are wound as as skein.

5. Device according to claim 2 **characterized in that** said fibres (71) are parallel to one another.

6. Device according to claim 1 **characterized in that** said haemo-concentrator (762) is positioned inside said casing (70), downstream to decapneization section (71).

7. Device according to claim 1 **characterized in that** said haemo-concentrator (762) consists of a hollow body positioned inside said casing (70) and containing high permeability hollow fibres (763).

8. Device according to claim 7 **characterized in that** said hollow fibres (763) are in polysulfone.

9. Device according to claim 7 **characterized in that** said hollow fibres (763) are parallel to one another.

10. Device according to claim 7 **characterized in that** said hollow fibres (763) are joined together by means of a potting in polycarbonate.

11. Device according to claim 7 **characterized in that** said hollow body of haemo-concentrator (762) is made ofpolyurethane.

12. Device according to claim 1 **characterized in that** said haemo-concentrator (762) is provided with an outlet section (764), which is connected to said outlet (75) for the blood, and with an outlet (767) for the ultrafiltrate.

13. Device according to claim 1 **characterized in that** said casing (70) is made of rigid polyurethane.

14. Device according to claim 1 **characterized in that** said casing has a basically cylindrical structure, a height (L) between 5 and 20 cm and a diameter (D) between 3,5 and 15 cm.

15. Device according to claim 1 **characterized in that** said casing has a basically cylindrical structure with a height (L) of about 10 cm and a diameter (D) of about 7 cm.

16. Device according to claim 1 **characterized in that** said second inlet (79) for the blood is positioned below said outlet (75) for the blood already treated.

17. Device according to claim 1 **characterized in that** the blood under treatment inside the device flows though a path from a downward direction to an upward direction.

18. Equipment for CRRT therapies **characterized in that** it is provided with a device for eliminating carbon dioxide from blood according to one of the previous claims.
